(19) Europäisches Patentamt
European Patent Office
Office européen des brevets

(11) Veröffentlichungsnummer : **0 011 254**
**B1**

(12) # EUROPÄISCHE PATENTSCHRIFT

(45) Veröffentlichungstag der Patentschrift : **13.01.82**

(51) Int. Cl.$^3$ : **C 07 C 89/00**, C 07 C 91/44, C 07 C 93/14

(21) Anmeldenummer : **79104418.3**

(22) Anmeldetag : **09.11.79**

(54) **Verfahren zur Herstellung von Hydroxy-diphenylaminen.**

(30) Priorität : **21.11.78 DE 2850391**

(43) Veröffentlichungstag der Anmeldung : **28.05.80 (Patentblatt 80/11)**

(45) Bekanntmachung des Hinweises auf die Patenterteilung : **13.01.82 Patentblatt 82/02**

(84) Benannte Vertragsstaaten : **CH DE FR GB**

(56) Entgegenhaltungen :
**DE - C - 601 996**
**GB - A - 349 677**
**US - A - 8 450 764**

(73) Patentinhaber : **BAYER AG**
**Zentralbereich Patente, Marken und Lizenzen**
**D-5090 Leverkusen 1, Bayerwerk (DE)**

(72) Erfinder : **Werner, Friedrich, Dr.**
**Petersenstrasse 1**
**D-5000 Köln 91 (DE)**
Erfinder : **Blank, Heinz Ulrich, Dr.**
**Am Geusfeld 35**
**D-5068 Odenthal (DE)**

**EP 0 011 254 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Verfahren zur Herstellung von Hydroxy-diphenylaminen

Die Erfindung betrifft ein Verfahren zur Herstellung von gegebenenfalls substituierten Hydroxy-diphenylaminen durch Kondensation eines gegebenenfalls substituierten Dihydroxybenzols mit einem gegebenenfalls substituierten primären aromatischen Amin.

Es ist aus der US-PS 3 450 764 bekannt, Dihydroxybenzole mit primären aromatischen Aminen in Gegenwart von para-Toluolsulfonsäure zu Diarylaminen zu kondensieren. Dieses Verfahren benötigt 9 bis 18 % para-Toluolsulfonsäure, bezogen auf das Dihydroxybenzol, und hat den verfahrenstechnischen Nachteil, daß vor der Isolierung des Reaktionsproduktes die gesamte Sulfonsäure durch eine aufwendige Extraktion isoliert werden muß.

Aus der DE-PS 601 996 ist ein Verfahren zur Kondensation von Phloroglucin und aromatischen Aminen in Gegenwart von Wasser bekannt, bei dem in Ausbeuten bis über 90 % der theoretischen Ausbeuten zwei Hydroxylgruppen durch Arylaminogruppen ersetzt werden. Die stark ausgeprägte Tendenz zum Ersatz auch der dritten Hydroxylgruppe kann insbesondere in Gegenart einer Säure nur durch die Begrenzung der Aminmenge zurückgedrängt werden. Zur Aufarbeitung wird das Reaktionsgemisch deutlich alkalisch gestellt, wobei das Kondensationsprodukt in Lösung geht und durch Säurebehandlung des alkalischen Filtrats ausgefällt wird.

Es wurde ein Verfahren zur Herstellung von Hydroxydiphenyl-aminen durch Kondensation eines Dihydroxybenzols mit einem Überschuß eines primären aromatischen Amins in Gegenwart von katalytischen Mengen einer Säure bei erhöhter Temperatur gefunden, das dadurch gekennzeichnet ist, daß man nach Beendigung der Kondensation das überschüssige primäre aromatische Amin und gegebenenfalls das Reaktionsprodukt in Gegenwart einer Base aus dem Reaktionsgemisch abdestilliert.

Als Dihydroxybenzole im erfindungsgemäßen Verfahren können beispielsweise solche der Formel (I)

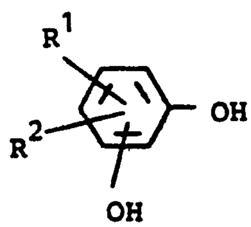

(I)

eingesetzt werden, in der

$R^1$ und $R^2$ gleich oder verschieden sein können und für Wasserstoff, Halogen, Alkyl, Cycloalkyl, Alkoxy, Phenyl, Phenyloxy, Cyano, die Carboxylgruppe, Alkoxycarbonyl, Alkylcarbonyloxy, Alkylcarbonyl oder die Sulfonsäureestergruppe steht,

wobei zusätzlich zur genannten Bedeutung $R^1$ und $R^2$, wenn sie benachbart sind, gemeinsam einen kondensierten cycloaliphatischen oder aromatischen Ring bilden können.

Bevorzugt können im erfindungsgemäßen Verfahren Dihydroxybenzol der Formel (I) eingesetzt werden, bei denen $R^1$ und $R^2$ gleich oder verschieden sein können und für Wasserstoff, Halogen, Alkyl, Alkoxy, Phenyl oder Phenyloxy stehen oder gemeinsam, wenn sie benachbart sind, einen kondensierten aromatischen Ring bilden können.

Besonders bevorzugt können im erfindungsgemäßen Verfahren Phenole der Formel (I) eingesetzt werden,

bei denen

$R^1$ und $R^2$ gleich oder verschieden sein können und für Wasserstoff, Halogen, Alkyl oder Alkoxy stehen oder gemeinsam, wenn sie benachbart sind, einen kondensierten aromatischen Ring bilden können.

Als primäre aromatische Amine können im erfindungsgemäßen Verfahren beispielsweise solche der Formel (II)

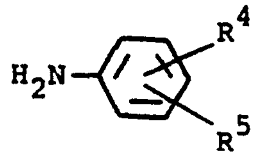

(II)

eingesetzt werden, in der

$R^4$ und $R^5$ gleich oder verschieden sein können und für Wasserstoff, Halogen, Alkyl, Cycloalkyl, Alkoxy, Phenyl, Phenyloxy, Cyano, die Carboxylgruppe, Alkoxycarbonyl, Alkylcarbonyloxy, Alkylcarbonyl,

oder die Sulfonsäure-estergruppe stehen, wobei zusätzlich $R^4$ und $R^5$, wenn sie benachbart sind, gemeinsam einen kondensierten cycloaliphatischen oder aromatischen Ring bilden können.

Bevorzugt können im erfindungsgemäßen Verfahren Amine der Formel (II) eingesetzt werden, bei denen

$R^4$ und $R^5$ gleich oder verschieden sein können und für Wasserstoff, Halogen, Alkyl oder Alkyloxy stehen, wobei zusätzlich $R^4$ und $R^5$, wenn sie benachbart sind, gemeinsam einen kondensierten aromatischen Ring bilden können.

Als Halogen seien beispielsweise Fluor, Chlor oder Brom, bevorzugt Chlor, genannt.

Als Alkyl seien beispielsweise geradkettige oder verzweigte Alkylreste mit 1 bis 4 Kohlenstoffatomen genannt, wie Methyl, Äthyl, Propyl, Isopropyl, Butyl oder Isobutyl. Bevorzugtes Alkyl ist Methyl oder Äthyl. Ganz bevorzugtes Alkyl ist der Methylrest.

Als Cycloalkyl seien beispielsweise carbocyclische Systeme mit 5 bis 7 Ringgliedern genannt, die gegebenenfalls durch niederes Alkyl substituiert sein können, wie Cyclopentyl, Methyl-Cyclopentyl, Cyclohexyl, Methyl-cyclohexyl, Cycloheptyl oder Methyl-Cycloheptyl. Bevorzugtes Cycloalkyl sind der Cyclopentyl- und der Cyclohexylring.

Als Alkoxy seien beispielsweise Reste genannt, die sich von einem niederen Alkohol mit 1 bis 4 Kohlenstoffatomen ableiten, wie Methoxy, Äthoxy, Propoxy, Isopropyloxy, Butoxy oder Isobutyloxy. Bevorzugtes Alkoxy ist der Methoxyrest.

Als Alkoxycarbonyl seien beispielsweise solche Reste genannt, die sich in der Estergruppe von einem niederen Alkohol mit 1 bis 4 Kohlenstoffatomen ableiten, wie der Methyl-, Äthyl-, Propyl- oder Butylester einer Carbonsäure.

Als Alkylcarbonyloxy seien beispielsweise eine durch eine $C_1$-$C_4$-Carbonsäure veresterte Hydroxylgruppe genannt, wie durch Ameisensäure, Essigsäure, Propionsäure oder Buttersäure veresterte Hydroxylgruppe.

Als Alkylcarbonyl seien beispielsweise Acylgruppen mit 2 bis 5 Kohlenstoffatomen genannt, wie Acetyl, Propionyl, Butyryl oder Valeroyl.

Als Sulfonsäureestergruppe seien beispielsweise solche genannt, die sich von einem niederen Alkohol mit 1 bis 4 Kohlenstoffatomen ableiten, wie der Sulfonsäuremethylester, -äthylester, -propylester oder -butylester.

Die Rester $R^1$ und $R^2$ und die Reste $R^4$ und $R^5$ können unabhängig voneinander, wenn sie benachbart sind, einen kondensierten cycloaliphatischen oder aromatischen Ring bedeuten. Der kondensierte cycloaliphatische Ring kann beispielsweise ein 5- oder 6-Ring sein. Auf dieser Weise kann man von der Benzolreihe in die Indanreihe, die Tetrahydronaphthalinreihe bzw. in die Naphthalinreihe gelangen.

Als Beispiele für Dihydroxybenzole, die im erfindungsgemäßen Verfahren eingesetzt werden können, seien genannt:

Brenzkatechin, Resorcin, Hydrochinon, alkylsubstituiertes Brenzkatechin, alkylsubstituiertes Resorcin, alkylsubstituiertes Hydrochinon, chlorsubstituiertes Brenzkatechin, chlorsubstituiertes Resorcin, chlorsubstituiertes Hydrochinon, 1,2-, 1,3-, 1,4-, 1,5-, 1,6-, 1,7- und 1,8-Dihydroxy-naphthalin, alkylsubstituiertes 1,2-, 1,3-, 1,4-, 1,5-, 1,6-, 1,7- und 1,8-Dihydroxy-naphtalin 2,3-Dihydroxy-benzoesäure, 2,4-Dihydroxy-benzoesäure, 2,5-Dihydroxy-benzoesäure, 2,3-Dihydroxy-benzoesäuremethylester, 2,4-Dihydroxy-benzoesäureäthylester, 2,5-Dihydroxy-benzoesäurebutylester, 2,3-, 2,4- und 2,5-Dihydroxydiphenyl.

Als Beispiele für primäre aromatische Amine, die im erfindungsgemäßen Verfahren eingesetzt werden können, seien genannt: Anilin, o-, m- und p-Chloranilin, o-, m- und p-Bromanilin, o-, m- und p-Toluidin, o-, m- und p-Cyclohexyl-anilin, o-, m- und p-Methoxy-anilin, o-, m- und p-Phenyl-anilin, o-, m- und p-Phenyloxy-anilin, 1-Naphthylamin, 2-Naphthylamin.

Das primäre aromatische Amin wird im erfindungsgemäßen Verfahren in einem molaren Überschuß, bezogen auf das eingesetzte Phenol, eingesetzt. Hierfür sei beispielsweise ein Verhältnis von 1,1 bis 10 Mol primäres aromatisches Amin auf 1 Mol Phenol genannt. Das bevorzugte Verhältnis beträgt 1,1 bis 7 Mol primäres aromatisches Amin, ganz besonders bevorzugt 1,2 bis 5 Mol primäres aromatisches Amin auf 1 Mol Phenol.

Die Kondensation im erfindungsgemäßen Verfahren wird in Gegenwart einer Säure der Formel (III)

$$R^6—SO_3H \qquad (III)$$

durchgeführt, in der

$R^6$ für Alkyl, Cycloalkyl, Aryl oder Hydroxyl steht.

Als Alkyl ($R^6$) sei beispielsweise ein geradkettiger oder verzweigter aliphatischer Kohlenwasserstoffrest mit 1 bis 18 Kohlenstoffatomen genannt, wie Methyl, Äthyl, Propyl, Isopropyl, Butyl, Isobutyl, Hexyl, Isohexyl, Octyl, Isodecyl, Dodecyl, Palmityl oder Stearyl.

Als Cycloalkyl ($R^6$) sei beispielsweise gegebenenfalls alkylsubstituiertes Cyclopentyl, Cyclohexyl oder Cycloheptyl genannt.

Als Aryl ($R^6$) sei beispielsweise ein gegebenenfalls substituierter aromatischer Rest aus der Benzol- oder Naphthalin-Reihe genannt, wie Phenyl, Toluyl, 1-Naphthyl oder 2-Naphthyl.

Als Beispiele für Säuren, die im erfindungsgemäßen Verfahren eingesetzt werden können, seien genannt: Methylsulfonsäure, Äthylsulfonsäure, Benzosulfonsäure, p-Toluolsulfonsäure, 1-Naphthylsulfonsäure, 2-Naphthylsulfonsäure, Schwefelsäure.

Die Säure der Formel (III) kann in katalytischer Menge im erfindungsgemäßen Verfahren eingesetzt werden. Als solche sei eine Menge von 1 bis 10 Gew.-%, bevorzugt 2 bis 5 Gew.-%, bezogen auf die eingesetzte Phenolmenge, genannt.

Die Kondensation im erfindungsgemäßen Verfahren kann in einem Temperaturbereich von 150 bis 250 °C, bevorzugt im Temperaturbereich von 180 bis 220 °C, durchgeführt werden. Ganz besonders bevorzugt ist die Durchführung der Kondensation in erfindungsgemäßen Verfahren beim Siedepunkt der am niedrigsten siedenden Reaktionskomponente.

Die Kondensation im erfindungsgemäßen Verfahren kann bei Normaldruck durchgeführt werden. Selbstverständlich ist es auch möglich, die Reaktion bei Überdruck durchzuführen, beispielsweise, um niedrig siedende Reaktionspartner im flüssigen Aggregatzustand zu halten. Hierzu sei ein Druck von bis zu 10 bar genannt. Bevorzugt wird unter dem Eigendruck der Reaktionskomponente gearbeitet, der sich bei der gewünschten Reaktionstemperatur einstellt.

Die Kondensation im erfindungsgemäßen Verfahren kann ohne Lösungsmittel durchgeführt werden. Selbstverständlich kann auch in Gegenwart eines in Bezug auf die Kondensation inerten Lösungsmittel gearbeitet werden. Als Beispiele für geeignete Lösungsmittel seien genannt: Aliphatische und aromatische Kohlenwasserstoffe, wie hochsiedende Benzinfraktionen oder Diphenyl, chlorierte Kohlenwasserstoffe, wie Dichlor-benzole, Diphenyläther, Sulfone und substituierte und unsubstituierte Säureamide.

Das bei der Kondensation im erfindungsgemäßen Verfahren entstehende Wasser kann kontinuierlich abdestilliert werden. Für den Fall, daß in Gegenwart eines Lösungsmittels gearbeitet wird, kann dies in Form einer Azeotrop-Destillation geschehen. Es ist jedoch auch möglich, die Entfernung des entstehenden Wassers aus dem Reaktionsgemisch mit Hilfe eines schwachen Inertgasstromes durch die Reaktionsmischung durchzuführen. Als Inertgas sei beispielsweise Stickstoff, Argon oder Kohlendioxid genannt.

Nach Abschluß der Kondensation kann das Reaktionsgemisch erfindungsgemäß durch Destillation in Gegenwart einer Base aufgearbeitet werden. Als Base seien beispielsweise die Hydroxide und Carbonate der Alkali- oder Erdalkalimetalle genannt, wie Natriumhydroxid, Kaliumhydroxid, Natriumcarbonat, Kaliumcarbonat, Magnesiumhydroxid, Calciumhydroxid, Magnesiumcarbonat oder Calciumcarbonat.

Die Base kann in fester oder gelöster Form eingesetzt werden, soweit es sich um lösliche Verbindungen handelt. Bei löslichen Basen verwendet man bevorzugt deren konzentrierte wäßrige Lösungen. Bevorzugt wird als Base Natriumhydroxid in Form von konzentrierter Natronlauge eingesetzt.

Selbstverständlich ist es auch möglich, organische Basen im erfindungsgemäßen Verfahren einzusetzen, wenn sie unter den Destillationsbedingungen nicht flüchtig sind.

Die Base wird in einer Menge zugesetzt, die ausreichend ist, um die im Reaktionsgemisch vorhandene Säure zu neutralisieren. Die Zugabe der erfindungsgemäß einzusetzenden Base kann vor Beginn der destillativen Auftrennung des Destillationsgemisches erfolgen. Es ist jedoch auch möglich, die Base während des Abdestillierens des im Überschuß eingesetzten primären aromatischen Amins zum Reaktionsgemisch zu geben.

Im Anschluß an das Abdestillieren des überschüssigen primären aromatischen Amins kann das als Reaktionsprodukt gebildete gegebenenfalls substituierte Hydroxy-diphenylamin, beispielsweise durch Destillation aus dem verbleibenden Rückstand oder durch Umkristallisation, gewonnen werden.

Im erfindungsgemäßen Verfahren können gegebenenfalls substituierte Hydroxy-diphenylamine der Formel

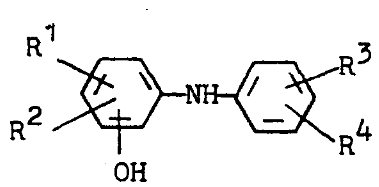

(IV)

in der $R^1$ bis $R^4$ die oben genannte Bedeutung haben, hergestellt werden.

Als Beispiele für Verbindungen der Formel (IV) seien genannt: 2-Hydroxy-diphenylamin, 3-Hydroxy-diphenylamin, 4-Hydroxy-diphenylamin, 2-Hydroxy-4-methyl-diphenylamin, 3-Hydroxy-4-chlor-diphenylamin, 2-Cyclohexyl-4-hydroxy-diphenylamin, 3-Hydroxy-4-äthoxy-diphenylamin, 2-Hydroxy-3-phenyl-diphenylamin, 3-Hydroxy-5-cyano-diphenylamin, 3-Hydroxy-diphenylamin-5-carbonsäuremethylester, 2-Hydroxy-diphenylamin-4-sulfonsäuremethylester, 3-Hydroxy-2'-methyl-diphenylamin, 3-Hydroxy-2'-chlor-diphenylamin, 3-Hydroxy-4'-methoxy-diphenylamin, 3-Hydroxy-4'-methyl-diphenylamin, 2-Hydroxy-4-phenyl-diphenylamin-4'-sulfonsäuremethylester, 3-Hydroxy-3-methoxy-5,5'-dicyano-diphenylamin, 1-(3-Hydroxy-naphthyl)-1-naphthyl-amin, 1-(2-Hydroxy-naphthyl)-4-methoxy-phenyl-amin, 1-(3-Hydroxy-5,6,7,8-tetrahydro-naphthyl)-phenyl-amin, 4-Hydroxy-phenyl-1-naphthyl-amin.

Das erfindungsgemäße Verfahren erlaubt in technisch fortschrittlicher Weise die Herstellung von

gegebenenfalls substituierten Diphenylaminen. Es zeichnet sich durch eine verfahrenstechnisch einfache Durchführung und hohe Raum/Zeit-Ausbeuten aus.

Es ist überraschend, daß das erfindungsgemäße Verfahren mit einfachen verfahrenstechnischen Maßnahmen fast quantitative Ausbeuten, bezogen auf eingesetztes Phenol, ergibt.

3-Hydroxy-diphenylamin kann in Gemisch mit 4-Chlor-2-aminophenol zum Färben von beispielsweise Pelzen, Haaren und Federn verwendet werden (DRP 334 011). Durch Kondensation von 2-Methyl-3-hydroxy-diphenylamin mit Phthalsäureanhydrid in Gegenwart von Schwefelsäure können Farbstoffe der Rhodaminreihe hergestellt werden (DRP 450 820). Weiterhin kann das Kaliumsalz des gegebenenfalls substituierten 3-Hydroxy-diphenylamins mit Kohlendioxid zu den entsprechenden 3-Hydroxy-diphenylamin-carbonsäuren umgesetzt werden (DRP 515 208).

## Beispiel 1

1 651,6 g (15 Mol) Resorcin werden mit 5 740,6 g (45 Mol) o-Chloranilin und 66,1 g p-Toluolsulfonsäure 18 Stunden bei 180 °C gerührt. Dabei gehen im schwachen $N_2$-Strom (10 $l/h^{-1}$) 84 g O-Chloranilin und 270 g (15 Mol) $H_2O$ über. In die warme Lösung werden 34,2 g Natronlauge 45 %ig = 15,4 g 100 % eingetropft. Dann destilliert man bei 1 mbar überschüssiges Chloranilin ab. Man gewinnt 3 405 g 2-Chlor-3'-hydroxydiphenylamin 96,7 %ig = 100 % der Theorie. Der Gehalt wurde gaschromatographisch nach der Methode des inneren Standards bestimmt.

## Vergleichsbeispiel 2 und Beispiele 3 bis 13

Analog Beispiel 1 wurden erhalten

| Beisp. | Einsatz (g) Resorcin | o-Chloranilin | Molverhältnis | Toluolsulfonsre (g) | Temp. °C |
|---|---|---|---|---|---|
| 2 | 1 651,6 | 5 740,6 | 1 : 3 | 66,1 | 180 |
| 3 | 1 651,6 | 5 740,6 | 1 : 3 | 66,1 | 180 |
| 4 | 552,0 | 1 278,0 | 1 : 2 | 22,0 | 180 |
| 5 | 552,0 | 767,0 | 1 : 1,2 | 22,0 | 180 |
| 6 | 552,0 | 1 918,0 | 1 : 3 | 22,0 | 190 |
| 7 | 552,0 | 1 278,0 | 1 : 2 | 22,0 | 190 |
| 8 | 552,0 | 960,0 | 1 : 1,5 | 22,0 | 190 |
| 9 | 552,0 | 767,0 | 1 : 1,2 | 22,0 | 190 |
| 10 | 552,0 | 1 918,0 | 1 : 3 | 22,0 | 200 |
| 11 | 552,0 | 1 278,0 | 1 : 2 | 22,0 | 200 |
| 12 | 552,0 | 960,0 | 1 : 1,5 | 22,0 | 200 |
| 13 | 552,0 | 767,0 | 1 : 1,2 | 22,0 | 200 |

| Beisp. | Natronlauge (g) | Zeit (h) | Gebildetes $H_2O$ (g) | 2-Chlor-3'-hydroxydiphenylamin (g) | Gehalt (%) | Ausbeute (%d.Th.) |
|---|---|---|---|---|---|---|
| 2 | — | 18 | 270 | 3 406,0 | 82,2 | 85,0 |
| 3 | 15,4 | 18 | 270 | 3 405,0 | 96,7 | 100,0 |
| 4 | 5,1 | 13 | 85 | 1 129,2 | 96,0 | 98,8 |
| 5 | 5,1 | 20 | 87 | 1 088,3 | 82,2 | 81,5 |
| 6 | 5,1 | 7 | 88 | 1 125,6 | 97,5 | 99,3 |
| 7 | 5,1 | 5,5 | 90 | 1 128,0 | 94,7 | 97,3 |
| 8 | 5,1 | 7,5 | 90 | 1 123,0 | 87,8 | 89,8 |
| 9 | 5,1 | 4 | 86 | 1 080,0 | 88,5 | 87,0 |
| 10 | 5,1 | 4 | 90 | 1 096,2 | 98,5 | 100,0 |
| 11 | 5,1 | 4 | 83 | 1 128,6 | 91,6 | 94,2 |
| 12 | 5,1 | 2,5 | 87 | 1 116,2 | 88,2 | 89,6 |
| 13 | 5,1 | 2,5 | 88 | 1 076,7 | 85,2 | 83,5 |

Beispiel 14

330 g Resorcin (3 Mol) werden mit 1 107 g 4-Methoxyanilin (9 Mol) und 13,2 g p-toluolsulfonsäure 18 Stunden bei 200 °C gerührt. Dabei destillieren 54 g Wasser ab. Man läßt auf 80 °C abkühlen und rührt 6,9 g Natronlauge 45 % ig ein. Dann wird überschüssiges 4-Methoxyanilin abdestilliert. Man gewinnt 658 g 3-Hydroxy-4'-methoxy-diphenylamin mit einer Reinheit von 95 % (= 96,9 % der theoretischen Ausbeute).

Beispiel 15

275 g Resorcin (2,5 Mol) werden mit 1 027,5 g 3-Methoxy-4-methylanilin (7,5 Mol) und 110 g p-Toluolsulfonsäure 21 Stunden bei 200 °C gerührt. Dabei destillieren 45 g $H_2O$ ab. Nach dem Abkühlen auf 70 °C werden 5,8 g Natronlauge 45 %ig eingerührt und das überschüssige 3-Methoxy-4-methylanilin abdestilliert. Man erhält 584 g 3-Hydroxy-3'-methoxy-4'-methyl-diphenylamin mit einer Reinheit von 93,5 % (= 95,4 % der theoretischen Ausbeute).

Beispiel 16

1 100 g Resorcin (10 Mol) werden mit 3 214,5 g o-Toluidin (30 Mol) und 44 g p-Toluolsulfonsäure 18 Stunden bei 200 °C gerührt. Dabei destillieren 180 g $H_2O$ ab. Nach dem Abkühlen werden 20,5 g Natronlauge 45 %ig eingerührt. und überschüssiges o-Toluidin abdestilliert. Man erhält 2 030 g 2-Methyl-3-hydroxy-diphenylamin mit einer Reinheit von 96 % (= 97,9 % der theoretischen Ausbeute).

Beispiel 17

330,3 g (3 Mol) Hydrochinon werden gemeinsam mit 838 g (9 Mol) Anilin und 13,2 g p-Toluolsulfonsäure unter Rühren auf 175 °C erhitzt. Die Temperatur wird mit fortschreitender Reaktion bis auf 205 °C gesteigert. Innerhalb von 35 Stunden destillieren 56 g $H_2O$ ab. Man läßt auf 80 °C abkühlen und rührt 6,9 g Natronlauge (45 Gew.-%) ein. Nach Abdestillieren des überschüssigen Anilins gewinnt man 567 g 4-Hydroxydiphenylamin mit einer Reinheit von 82,8 Gew.-% ; das entspricht 87,5 % der theoretischen Ausbeute.

**Ansprüche**

1. Verfahren zur Herstellung von Hydroxy-diphenylaminen durch Kondensation eines Dihydroxybenzols mit einem Überschuß eines primären aromatischen Amins in Gegenwart von katalytischen Mengen einer Säure bei erhöhter Temperatur, dadurch gekennzeichnet, daß man nach Beendigung der Kondensationsreaktion das überschüssige primäre aromatische Amin und gegebenenfalls das Reaktionsprodukt in Gegenwart einer Base aus dem Reaktionsgemisch abdestilliert.

2. Verfahren nach Anspruch 1, dadurch gekennzeichnet, daß man als Base das Hydroxid oder Carbonat eines Alkali- oder Erdalkalimetalls einsetzt.

3. Verfahren nach Anspruch 1 und 2, dadurch gekennzeichnet, daß man als Base konzentrierte Natronlauge einsetzt.

**Claims**

1. Process for the preparation of hydroxy-diphenylamines by condensation of a dihydroxybenzene with an excess of a primary aromatic amine in the presence of catalytic amounts of an acid at elevated temperature, characterised in that when the condensation reaction has ended, the excess primary aromatic amine and if appropriate the reaction product are distilled off from the reaction mixture in the presence of a base.

2. Process acccording to Claim 1, characterised in that the hydroxide or carbonate of an alkali metal or alkaline earth metal is employed as the base.

3. Process according to Claim 1 and 2, characterised in that concentrated sodium hydroxide solution is employed as the base.

**Revendications**

1. Procédé de fabrication d'hydroxydiphénylamines par condensation d'un dihydroxybenzène avec un excès d'une amine aromatique primaire en présence de quantités catalytiques d'un acide à haute température, caractérisé en ce qu'après l'achèvement de la réaction de condensation on sépare par distillation l'amine aromatique primaire en excès et éventuellement le produit de réaction en présence d'une base à partir du mélange de réaction.

2. Procédé selon la revendication 1, caractérisé en ce qu'on utilise en tant que base l'hydroxyde ou le carbonate d'un métal alcalin ou alcalino-terreux.

3. Procédé selon les revendications 1 et 2, caractérisé en ce qu'on utilise comme base de la soude caustique concentrée.